# EUROPEAN PATENT SPECIFICATION

(11) **EP 2 331 180 B1**
(45) Date of publication and mention of the grant of the patent: **20.12.2017**
(21) Application number: 09769731.2
(22) Date of filing: 18.06.2009
(51) Int. Cl.: A61M 16/04

(54) **TRACHEOSTOMY TUBE**
TRACHEOSTOMIE-TUBUS
TUBE DE TRACHÉOSTOMIE

(30) Priority: 27.06.2008 US 163173
(43) Date of publication of application: 15.06.2011
(73) Proprietor: Avent, Inc., Alpharetta, GA 30004 (US)
(72) Inventor: KENOWSKI, Mike, Alpharetta GA 30005 (US); SCHUMACHER, James, Cumming GA 30041 (US)
(74) Representative: Davies, Christopher Robert
(86) International application number: PCT/IB2009/052625
(87) International publication number: WO 2009/156922

(56) References cited:
- EP-A1- 1 854 499
- EP-A2- 0 546 712
- WO-A1-93/24170
- WO-A1-99/34856
- WO-A1-2004/101046
- GB-A- 2 306 112
- GB-A- 2 409 163
- US-A- 5 515 844

## Description

Ventilators or respirators are used for mechanical ventilation of the lungs of a patient in a medical setting. The ventilator unit is connected to a hose set; the ventilation tubing or tubing circuit, delivering the ventilation gas to the patient. At the patient end, the ventilation tubing is typically connected to a tracheal ventilation catheter or tube, granting direct and secure access to the lower airways of a patient. Tracheal catheters are equipped with an inflated sealing balloon element, or "cuff', creating a seal between the tracheal wall and tracheal ventilation tube shaft, permitting positive pressure ventilation of the lungs.

One type of tracheal catheter, an endotracheal tube (ET tube), inserted through the mouth, is generally used for a number of days before a decision is made to switch a patient to a tracheostomy tube, inserted directly into the trachea through a stoma in the tracheal wall. Endotracheal tubes have been linked in some studies to an increased rate of ventilator acquired pneumonia (VAP) and so tracheostomy operations are becoming increasingly common and are being performed earlier in the patient's hospital stay in order to reduce the occurrence of VAP.

A tracheostomy procedure involves making a small horizontal incision in the skin of the neck to grant access to the trachea. Because of the uniquely flexible and elastic nature of the trachea, it has been found that healing is much faster if only a small hole is made in the tracheal wall and the hole dilated, rather than cutting the tracheal wall. After the trachea has been dilated, a tracheostomy or "trach" tube is inserted through the stoma, the balloon cuff inflated and the trach tube connected to a ventilator.

The amount of force needed to insert a trach tube into the trachea can cause the tubes to kink and collapse. Great care is needed to avoid this problem, lengthening the time necessary to perform this procedure.

WO93/24170 discloses a tracheal tube with the features of the preamble of claim 1.

US 5515844 discloses a tracheostomy tube reinforcing wire embedded along the whole tube.

GB 2409163 discloses a laryngeal mast partially reinforced by wire.

There remains a need for a device that can more quickly and safely allow for the successful placement of a tracheostomy tube.

### SUMMARY OF THE INVENTION

According to the invention, there is provided a tracheostomy tube as set forth in claim 1. This novel tracheostomy tube largely overcomes the problem of trach tube collapse. The tube has a variable flexibility. The tube is flexible at its distal portion so as to pose less of a problem for the posterior wall of the trachea should it contact it. It is less flexible at its proximal portion where the greatest amount of force is generally applied during a tracheostomy procedure and after placement due to the tracheal rings. The upper or proximal portion is as much as two thirds of the length of the tube between the flange and the start of the sealing cuff. The lower or distal portion is the balance of the tube between the start of the sealing cuff and the distal end.

### BRIEF DESCRIPTION OF THE DRAWINGS

Figure 1 is a drawing of the trachestomy tube 26 with the cannula removed
Figure 2 is a drawing of the loading catheter 50 installed in the trach tube 26.
Figure 3 is a drawing of the trach tube in its final position in the trachea, with the trach cuff inflated.
Figure 4 is a drawing of the replaceable (disposable) cannula for use with the trach tube.
Figure 5 is a drawing of the trach tube showing the removable cannula installed in the tube.
Figure 6 is a drawing of a trach tube having reinforcing wire on the upper portion of the shaft.
Figure 7 is a drawing of a trach tube with reinforcing rods along the exterior of the upper portion of the shaft.
Figure 8 is a drawing of the cross-section of the reinforcing rods shown in Figure 7 with hollow lumens within thickness of the rods.
Figure 9 is a drawing of a trach tube with concentric reinforcing rings along the exterior of the upper portion of the shaft.

### DETAILED DESCRIPTION OF THE INVENTION

Tracheostomy is a lifesaving procedure to allow a patient to be ventilated directly through the trachea. Tracheostomy is also believed by many to prevent or delay the onset of ventilator acquired pneumonia (VAP). This lifesaving procedure, unfortunately, is relatively time consuming and current technology requires a large number of steps and pieces of equipment that must remain sterile and functioning properly in order to arrive at a successful conclusion. The tracheostomy procedure may be greatly improved using tapered dilators and trach tube loading catheters or obturators.

Dilators are instruments or substances for enlarging a canal, cavity, blood vessel or opening, according to the American Heritage Stedman's Medical dictionary 2001. Once a dilator is used to enlarge the stoma in the trachea for placement of the trach tube, the tube is inserted to the point at which the flange 70 touches the skin of the patient.

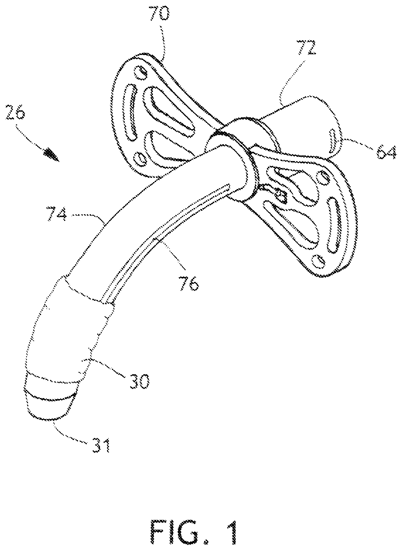

The tracheostomy tube is shown in Figure 1. The tube has a flange 70 on or near the proximal end for attachment to the patient's skin using holes 71 for suturing that may be located at the corners of the flange. The tube 26 has a proximal end 72 for attachment to a ventilator once the tube is in place in the trachea. The tube has a location for attachment of an inflation line 76 so that a pressurizing gas, generally air, may be supplied to a balloon cuff 30 near the distal end of the trach tube 26. The upper portion; one third to two thirds of the shaft 74 of the tube, extending from below (distal to) the flange in the distal direction, is the area of highest stress when a tube is inserted. While the entire tube may be reinforced if desired according to this disclosure, reinforcement of the upper portion of the tube provides for a tube that may be more successfully placed while also providing a less traumatic lower portion that may contact the posterior of the trachea.

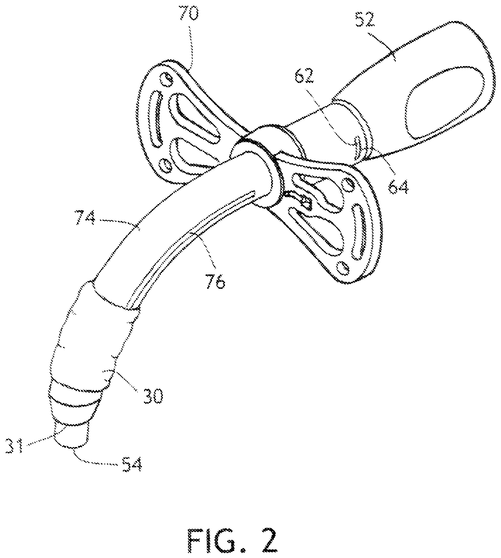

In order to place a trach tube in the trachea of a patient, a loading catheter 50 is desirably slid into the tracheostomy tube 26 (Figure 2) prior to insertion. The loading catheter handle 52 detachably engages the proximal end of the trach tube 26 with, for example, a slot 64 and tab 62 arrangement. There may also be tabs 62 on both sides of the handle 52 which mate with slots 64 on the proximal end of the trach tube 26. Once engaged, the handle is desirably not freely rotational. Those skilled in the art may easily devise alternative ways of mating the handle 52 with the tube 26.

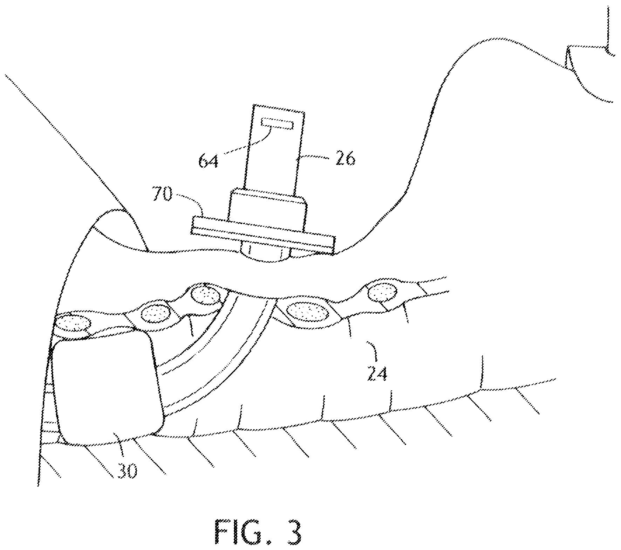

The tracheostomy tube 26 with the loading catheter 50 inside (Figure 2) may be inserted into the trachea, optionally with the assistance of a dilator pursuant to the patent application filed the same day as this application by the same assignee and entitled "Easy Grip Tapered Dilator". Once the tube 26 is in place in the trachea, the loading catheter 50 and any other removable parts may be withdrawn through the tracheostomy tube 26 with only the tube 26 remaining in place in the trachea 24 (Figure 3).

The loading catheter 50 may be removed from the trach tube by disengaging the detachably attached handle 52 from the proximal end of the tracheostomy tube 26 and pulling the handle 52 away from the tube 26. One way of accomplishing this disengagement is by twisting the loading catheter handle 52. This twisting action cams the loading catheter handle 52 off the proximal end of the trach tube 26, overcoming any static friction that may exist in the system and defeating the tabs 62 and slots 64 locking the loading catheter handle 52 to the tube 26. This action allows the user to pull all the loading components out through the inner lumen of the trach tube 26, leaving only the tube 26 in place. Clearly the optionally dilator tip 12 must be sized so that its largest diameter is slightly less than that of the tracheostomy tube 26 that it is intended to pass through. Once the trach tube 26 is in place, the tube cuff 30 is inflated and the tube 26 is connected to a ventilator (not shown) and placed in service (Figure 3).

The trach tube 26 has a balloon cuff 30 around its circumference on a lower (distal) portion of the tube that serves to block the normal air flow in the trachea so that (assisted) breathing takes place through the trach tube using a ventilator. The cuff is desirably made from a soft, pliable polymer such as polyurethane (PU), polyethylene teraphihalate (PETP), low-density polyethylene (LDPE), polyvinyl chloride (PVC), or elastomeric-based polyolefins. It should be very thin; on the order of 25 microns or less, e.g. 20 microns, 15 microns, 10 microns or even as low as 5 microns in thickness. The cuff should also desirably be a low pressure cuff operating at about 30 mmH₂O or less, such as 25 mmH₂O, 20 mmH₂O, 15 mmH₂O or less. Such a cuff is described in US patent 6,802,317 which describes a cuff for obturating a patient's trachea as hermetically as possible, comprising a cuffed balloon which blocks the trachea below a patient's glottis, an air tube, the cuffed balloon being attached to the air tube and being sized to be larger than a tracheal diameter when in a fully inflated state and being made of a soft, flexible foil material that forms at least one draped fold in the cuffed balloon when inflated in the patient's trachea, wherein the foil has a wall thickness below or equal to 0.01 mm and the at least one draped fold has a loop found at a dead end of the at least one draped fold, that loop having a small diameter which inhibits a free flow of secretions through the loop of the at least one draped fold. Another description of such a cuff is in US patent 6,526,977 which teaches a dilator for obturating a patient's trachea as hermetically as possible, comprising a cuffed balloon which blocks the trachea below a patient's glottis, an air tube, the cuffed balloon being attached to the air tube and being sized to be larger than a tracheal diameter when in a fully inflated state and being made of a sufficiently soft, flexible foil material that forms at least one draped fold in the cuffed balloon when fully inflated in the patient's trachea, wherein the at least one draped fold formed has a capillary size which arrests free flow of secretions across the balloon by virtue of capillary forces formed within the fold to prevent aspiration of the secretions and subsequent infections related to secretion aspiration.

There is a flange 70 on the trach tube 26 on the proximal end that is used to attach the trach tube to a patient's throat. The flange 70 extends on either side of the tube 26 near the proximal end where the ventilator connection 72 is located. The flange 70 is flexible and non-irritating and can be sutured onto the throat of a patient to anchor the tube 26. The size of the flange will vary depending on the size and needs of the patient.

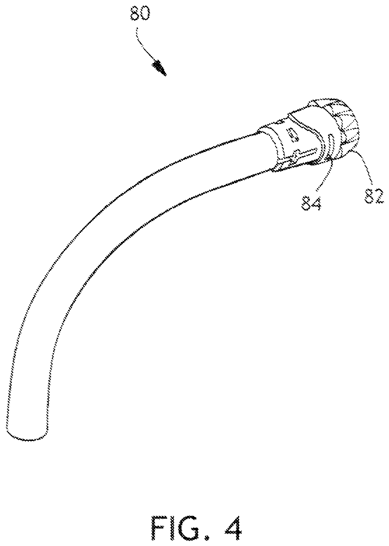

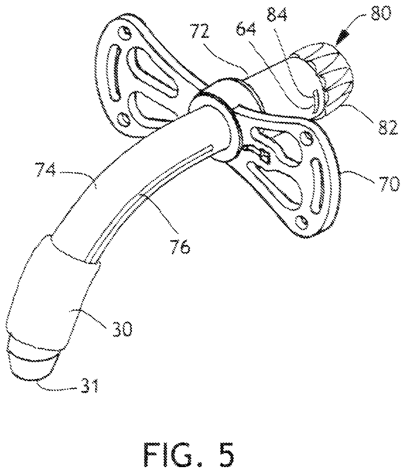

The trach tube 26 also may be used with disposable cannulas 80 (Figure 4) that are placed within the trach tube from the proximal end (Figure 5) These disposable cannulas 80 are changed regularly so that bacterial growth is kept to a minimum. The cannulas are made from a plastic material such as a polyolefin, polyurethane, nylon, etc and are desirably semi-rigid. Cannulas may be treated with anti-bacterial and/or anti-viral coatings or other active materials to help reduce the growth of harmful organisms. The cannula 80 may be attached to the trach tube 26 in a manner similar to the attachment of the loading catheter 50, i.e., using tabs 84 on the proximal end 82 that mate with the slots 64 on the tube.

The flange 70 may desirably be of a width between 6 and 12 cm and height of 1 to 6 cm, more particularly between 7 and 10 cm and 2 and 5 cm respectively or still more particularly between 8 and 9 cm and 2 and 4 cm respectively. The distance from the flange 70 to the distal tip 31 of the trach tube 26 may be an arched distance of between 70 and 100 mm, desirably between about 75 and 95 mm and more desirably between 80 and 90 mm. The angle of the trach tube from the flange to the distal end is between 85 and 120 degrees, desirably between 95 and 115 degrees, more desirably between 100 and 110 degrees. Materials that are suitable for making a trach tube and flange include polyurethanes, polyvinyl chlorides, nylons, polyolefins and other biocompatible polymers. Depending on the polymer chosen, the trach tube and flange may be transparent, translucent or opaque.

One way of enhancing the strength of the upper portion of the trach tube shaft 74 is to make the shaft of materials of different hardnesses. One suitable measurement of hardness known to those skilled in the art is the Durometer ASTM D2240 hardness test,. The upper portion of the shaft may be made, for example, of a relatively harder polymer than the lower portion of the shaft. The use of the same type of material, e.g., polyurethane, allows the polymers to be fashioned into a tube in the same manner and at nearly the same conditions, and helps ensure a strong and seamless transition. The upper portion of the shaft may be made, for example, by injection molding a 55 D Shore hardness polyurethane while the lower portion is injection molded, simultaneously in the same mold, of an 80 A Shore hardness polyurethane. The resulting shaft with vary in flexibility.

Rather than making the upper and lower portion of the trach tube from different hardness polymers, a variable blend of polymers may be used with a greater proportion of a harder polymer in the upper portion, gradually tapering to a much lower amount in the lower portion of the tube. Again, this may be accomplished by using an injection molding procedure as is known to those skilled in the art.

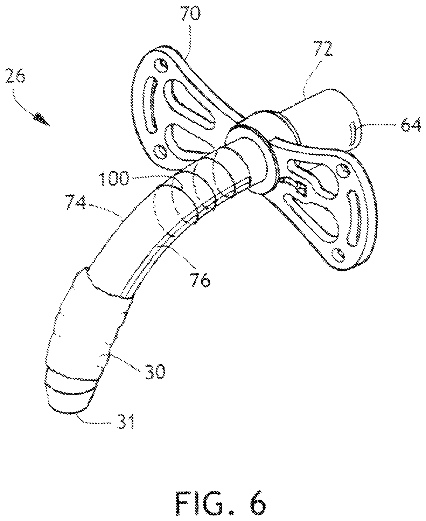

Another way to enhance the strength of the tube, outside the scope of the invention, is by winding wire 100 about the tube (Figure 6). Unfortunately, metallic wire may interfere with X-ray, MRI or other scanning procedures so, if the wire is metallic, it should be installed beneath the interior and surface of the tube shaft so that it is completely encapsulated by the tube in order to avoid exposing a patient to contact with the metal. Metals suitable for use in this embodiment include titanium, cobalt, stainless steel and the like.

Plastic wire reinforcement may be provided on the exterior shaft surface but in such a position may make it more difficult to insert the tube as it may catch on the edge of the tracheal stoma as the tube is being inserted. Suitable plastics include polytetrafluoroethylene (PTFE) or fluoroethylene propylene (FEP) and other relatively high melt temperature materials. The use of wire reinforcement, of any type, would permit the tube shaft 74 to be made of only one uniform type of material. A prefabricated wire may be slid over the trach tube after fabrication. Alternatively the wire could be installed in the trach tube mold prior to injection so that it becomes imbedded in the tube as it is produced. Wire reinforcement may be added to the upper portion of the shaft using between 3 and 20 windings per inch (1.2 to 8 windings per cm), desirably between 5 and 10 windings per inch (2 and 4 windings per cm).

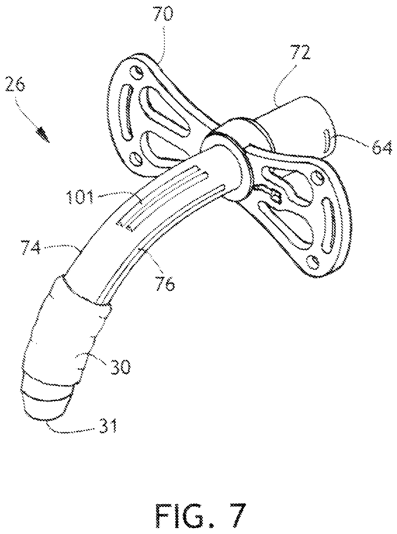

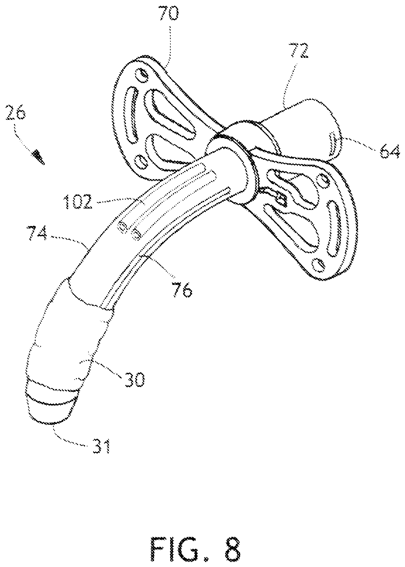

Another way to enhance the strength of a tracheostomy tube, which again is outside the scope of the present invention, is to place reinforcing rods 101 along the sides of the tube (Figure 7) at various positions around the circumference of the tube. The rods may be made from a less flexible material than the trach tube and attached after manufacture of the tube. Alternatively the rods may be injection molded with the tubes using a polymer of a greater hardness than the tube. The rods 101 could also contain lumens 103 within the walls that could provide transport of air or liquids for such functions as inflating the balloon cuff or managing fluid secretions along the shaft both above and below the balloon cuff, as shown in Figure 8.

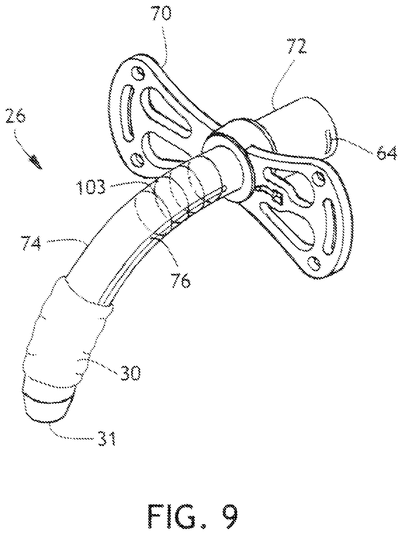

Yet another way to enhance the strength of the tube, outside the scope of the invention, is to install concentric rings 102 around the tube (Figure 9). Like the rods, the rings may be added to the tube after manufacture or injection molded with the tubes. The rings 102 may be but need not necessarily be of a greater hardness than the tubes since their shape adds considerably to the strength of the tube without consideration of polymer type. The rods 101 and rings 102 may be from 1 to 8 mm in width.

This application is one of a group of commonly assigned patent application which are being filed on the same day. The group includes application serial no.:12/147,817 in the name of Brian Cuevas and is entitled "Easy Grip Tapered Dilator"; application serial no.:12/147,873 in the name of Brian Cuevas and is entitled "Method of Performing a Tracheostomy"; application serial no.: 12/163,065 in the name of Michael Sleva and is entitled "Dilator Loading Catheter"; application serial no.:12/147,952 in the name of Brian Cuevas and is entitled "Tracheostomy Tube Butterfly Flange"; application serial no.: 12/163,173 in the name of James Schumacher and is entitled "Tracheostomy Tube"; design application no. 29/320,497 in the name of Brian Cuevas and is entitled "Butterfly Flange"; design application serial no. 29/320,492 in the name of Brian Cuevas and is entitled "Tapered Dilator Handle"; design application 29/320,500 in the name of Brian Cuevas and is entitled "Stoma Pad".

As will be appreciated by those skilled in the art, changes and variations to the invention are considered to be within the ability of those skilled in the art. Such changes and variations are intended by the inventors to be within the scope of the invention. It is also to be understood that the scope of the present invention is not to be interpreted as limited to the specific embodiments disclosed herein, but only in accordance with the appended claims when read in light of the foregoing disclosure.

## Claims

1. A tracheostomy tube (26) comprising a tube (74) with a cannula and a flange (70) near a proximal end (72), said tube (74) having an upper portion and a distal portion wherein said upper portion is reinforced; wherein said upper portion extends at most two thirds of a distance along said tube (74) in a direction distal from said flange (70) toward a distal end (31) of said tube (74), **characterised in that** said distal portion has a lesser hardness than said upper portion.

2. The tracheostomy tube (26) of claim 1 wherein said distal portion is made from a polymer having a lower hardness that a polymer used to make said upper portion.

3. The tracheostomy tube (26) of claim 1 wherein said tracheostomy tube is made from a blend of polymers having different hardnesses.

4. The tracheostomy tube (26) of claim 1 comprising a flexible flange (70).

5. The tracheostomy tube (26) of claim 1 comprising a balloon cuff (30) made from a soft, pliable polymer and having a thickness between 5 and 25 microns.

6. The tracheostomy tube (26) of claim 5 wherein said polymer is polyurethane.

7. The tracheostomy tube (26) of claim 6 wherein said tube (74) has an arched distance of between 70 and 100 mm from said flange (70) to said distal end (31).

8. The tracheostomy tube (26) of claim 6 wherein said tube (74) has an arched distance of between 75 and 95 mm from said flange (70) to said distal end (31).

9. The tracheostomy tube (26) of claim 6 wherein said tube (74) has an arched distance of between 80 and 90 mm from said flange (70) to said distal end (31).

10. The tracheostomy tube (26) of claim 1 wherein an angle of the trach tube (26) from the flange (70) to the distal end (31) is between 85 and 120 degrees, preferably between 95 and 115 degrees, and more preferably between 100 and 110 degrees.

## Patentansprüche

1. Tracheostomietubus (26), umfassend einen Tubus (74) mit einer Kanüle und einem Flansch (70) in Nähe eines proximalen Endes (72), wobei der Tubus (74) einen oberen Abschnitt und einen distalen Abschnitt aufweist, wobei der obere Abschnitt verstärkt ist; wobei der obere Abschnitt sich höchstens über zwei Drittel eines Abstands entlang dem Tubus (74) in einer Richtung distal von dem Flansch (70) zu einem distalen Ende (31) des Tubus (74) hin erstreckt, **dadurch gekennzeichnet, dass** der distale Abschnitt eine geringere Härte als der obere Abschnitt aufweist.

2. Tracheostomietubus (26) nach Anspruch 1, wobei der distale Abschnitt aus einem Polymer hergestellt ist, das eine niedrigere Härte als ein zur Herstellung des oberen Abschnitts verwendetes Polymer aufweist.

3. Tracheostomietubus (26) nach Anspruch 1, wobei der Tracheostomietubus aus einem Gemisch von Polymeren hergestellt ist, die unterschiedliche Härten aufweisen.

4. Tracheostomietubus (26) nach Anspruch 1, welcher einen flexiblen Flansch (70) umfasst.

5. Tracheostomietubus (26) nach Anspruch 1, welcher eine Ballonmanschette (30) umfasst, die aus einem weichen, biegsamen Polymer hergestellt ist und eine Dicke zwischen 5 und 25 Mikron aufweist.

6. Tracheostomietubus (26) nach Anspruch 5, wobei das Polymer Polyurethan ist.

7. Tracheostomietubus (26) nach Anspruch 6, wobei der Tubus (74) einen gewölbten Abstand von zwischen 70 und 100 mm von dem Flansch (70) bis zu dem distalen Ende (31) aufweist.

8. Tracheostomietubus (26) nach Anspruch 6, wobei der Tubus (74) einen gewölbten Abstand von zwischen 75 und 95 mm von dem Flansch (70) bis zu dem distalen Ende (31) aufweist.

9. Tracheostomietubus (26) nach Anspruch 6, wobei der Tubus (74) einen gewölbten Abstand von zwischen 80 und 90 mm von dem Flansch (70) bis zu dem distalen Ende (31) aufweist.

10. Tracheostomietubus (26) nach Anspruch 1, wobei ein Winkel des Trachealtubus (26) von dem Flansch (70) bis zu dem distalen Ende (31) zwischen 85 und 120 Grad, bevorzugt zwischen 95 und 115 Grad, und bevorzugter zwischen 100 und 110 Grad beträgt.

## Revendications

1. Tube de trachéostomie (26) comprenant un tube (74) avec une canule et une collerette (70) près d'une extrémité proximale (72), ledit tube (74) ayant une portion supérieure et une portion distale, dans lequel ladite portion supérieure est renforcée ; dans lequel ladite portion supérieure s'étend au plus sur les deux tiers d'une distance le long dudit tube (74) dans une direction distale par rapport à ladite collerette (70) vers une extrémité distale (31) dudit tube (74), **caractérisé en ce que** ladite portion distale a une dureté inférieure à ladite portion supérieure.

2. Tube de trachéostomie (26) selon la revendication 1, dans lequel ladite portion distale est faite d'un polymère ayant une dureté inférieure à un polymère utilisé pour faire ladite portion supérieure.

3. Tube de trachéostomie (26) selon la revendication 1, dans lequel ledit tube de trachéostomie est fait d'un mélange de polymères ayant différentes duretés.

4. Tube de trachéostomie (26) selon la revendication 1, comprenant une collerette flexible (70).

5. Tube de trachéostomie (26) selon la revendication 1, comprenant un manchon de ballonnet (30) fait d'un polymère pliable, souple et ayant une épaisseur entre 5 et 25 microns.

6. Tube de trachéostomie (26) selon la revendication 5, dans lequel ledit polymère est du polyuréthane.

7. Tube de trachéostomie (26) selon la revendication 6, dans lequel ledit tube (74) a une distance d'arc entre 70 et 100 mm de ladite collerette (70) à ladite extrémité distale (31).

8. Tube de trachéostomie (26) selon la revendication 6, dans lequel ledit tube (74) a une distance d'arc entre 75 et 95 mm de ladite collerette (70) à ladite extrémité distale (31).

9. Tube de trachéostomie (26) selon la revendication 6, dans lequel ledit tube (74) a une distance d'arc entre 80 et 90 mm de ladite collerette (70) à ladite extrémité distale (31).

10. Tube de trachéostomie (26) selon la revendication 1, dans lequel un angle du tube de trachéostomie (26) de la collerette (70) à l'extrémité distale (31) est entre 85 et 120 degrés, préférablement entre 95 et 115 degrés, et plus préférablement entre 100 et 110 degrés.
